# EUROPEAN PATENT SPECIFICATION

(11) **EP 0 679 892 B1**
(45) Date of publication and mention of the grant of the patent: **28.08.2002**
(21) Application number: 95106506.9
(22) Date of filing: 28.04.1995
(51) Int. Cl.: G01N 33/546

(54) **Microparticle immunoassay reagents, sensitive and specific immunoassay reagents and immunoassay methods using these reagents**
Mikropartikel-Immunoassayreagentien, empfindliche und spezifische Immunoassayreagentien und Immunoassaymethode
Réactifs d'essais immunologiques à micro-particules, réactifs d'essais immunologiques spécifiques et méthodes d'essais immunologiques utilisant ces réactifs

(30) Priority: 29.04.1994 US 235785; 02.05.1994 US 235876
(43) Date of publication of application: 02.11.1995
(73) Proprietor: Seradyn, Inc., Indianapolis, IN 46268 (US)
(72) Inventor: Yanagida, Atsushi, Indianapolis, IN 46280 (US); Sugawa, Satoshi, c/o Mitsubishi Chem. Corp., Aoba-ku, Yokohama, Kanagawa (JP); Ito, Michio, c/o Mitsubishi Chem. Corp., Chiyoda-ku, Tokyo (JP)
(74) Representative: TER MEER STEINMEISTER & PARTNER GbR

(56) References cited:
- WO-A-90/08321
- US-A- 5 302 532

## Description

### BACKGROUND OF THE INVENTION

### Field of the Invention

The present invention relates to microparticle immunoassay reagents. More particularly, the present invention relates to immunoreactant-sensitized carboxylate-modified latex microparticles having high sensitivity and good dispersibility. The invention microparticle reagents are useful in all particle-based immunoreactant assays and detection systems.

The present invention also relates to highly sensitive and specific immunoreactant assay reagents. More particularly, the present invention relates to assay reagents and systems wherein a sensitized solid reagent is provided and used in combination with a particular buffer reagent that reduces non-specific antigen-antibody reactions. The assay reagents of the present invention are designed to avoid the need for sample pretreatment and be compatible with commercial autoanalyzers typically used in the field of clinical chemistry.

### Discussion of the Background

Particle-based immunoassays are well-known, and they constitute a significant portion of the immunoassay market. This is because particle-based immunoassays are highly versatile, and they may be used in tests requiring only simple visual detection and in quantitative, instrument-based analytical methods. For example, slide latex agglutination methods are known wherein an antibody or antigen is bound to latex particles and used for the detection of an antigen or antibody, respectively. In either situation, the binding of the antibody or antigen to the latex particles is typically accomplished by passive adsorption or covalent coupling.

Particle agglutination methods may be used to directly detect an antigen or an antibody (the latter being an indirect indication of the presence of an antigen). Endpoints are detected visually or instrumentally, and are chosen as either agglutination or agglutination inhibition states depending up on the specific analyte being tested. Slide tests using latex agglutination methods are typically utilized in a positive/negative qualitative format and are commonly used for infectious disease assays. Microparticles sensitized with an immunoreactant (i.e., an antigen or an antibody) have also found use in radioimmunoassays, enzyme linked immunosorbent assays (ELISA, and membrane or particle capture-type assays.

While visual detection (agglutination, color development, etc.) is satisfactory for general screening or yes/no qualitative investigations, certain analytes require quantitative assay (drugs, haptens, etc.). Moreover, when a particle-based assay is used in high-volume screening applications (e.g., AIDS, hepatitis, etc.), it is desirable to have a fully-automated method. Particle-based instrument assays are currently being developed to meet this need. These instruments and their attendant methods are typically set up for the direct detection of an immunoreactant, optionally with multiple epitopes, or as inhibition methods for, e.g., haptens (a small molecule bound to a larger one; e.g., a drug-protein conjugate).

Several instruments and methods have been developed to quantitate immunoreactant concentration in an unknown sample by utilizing the changes occurring in light scattering caused by the specific reaction of sensitized particles with immunoreactants. Due to the large surface area of particle suspensions and the physical principles of light scattering, such methods and instrumentation can easily discriminate between signals varying from one another by from 2-3%, whereas the semi-quantitative visual evaluation methods described above have difficulty discriminating between 30-40% differences.

Immunoassay methods and devices like those described above are explained in the Handbook of Experimental Immunology, vols. 1-4, Blackwell Scientific 1987, in U.S. Patents 3,088,875, 3,857,931, 3,992,517, 4,080,264, 4,174,952, 4,203,724, 4,480,042, 4,590,156, 4,690,906, 4,716,123, 4,772,550, 4,851,329, 4,960,692 and 5,100,805, and in Grange, J. et al., J. Immuno. Meth., 18,365,1977, Hechemy, K. et al., Lab Management, 27, June/July 1984, Looney, C., J. Clin. Immunoassay, 7,(1), 90, 1984, Von Schulthes, G. et al., Mol Immunol, 1, 81, 1980, Craine, J., Am. Biotech, Lab., 34, May-June, 1987, Heveran, J., J. Forensic Sci., 470, 1977 and Kimura, H. J. Immuno. Meth., 38, 353, 1980.

While particle-based immuoassays and sensitized (i.e., immunoreactant coated- or modified-) microparticle reagents are known, current microparticles lack the desirable characteristics of high sensitivity, good dispersibility and stability.

WO-A-90/08321 discloses submicron particles for the use in immunodiagnosis wherein an immunoreactant is covalently bound to particles of acrylic latex having a mean diameter lower than or equal to 0.1µm, wherein said latex includes 100-450 microequivalents of COOH groups per gram of latex.

Digoxin is a popular cardiac glycoside currently prescribed for the control of congestive heart failure and for certain cardiac rhythm abnormalities. The increased cardiac output resulting from the inotropic action of digoxin ameliorates the disturbances characteristic of heart failure such as venous congestion, edema, dyspnea, orthopnea and cardiac asthma. Digoxin also reduces ventricular rate and thus improves hemodynamics. Palpitation, precordial distress or weakness are relieved and concomitant congestive failure ameliorated. Digoxin also slows the heart and induces regular sinus rhythm. Regardless whether digoxin is used to control/inhibit heart failure, atrial fibrillation or flutter, the cotinued administration of digoxin after the onset of clinical event is typically recommended.

The therapeutic index for digoxin is very low, there being only a very narrow difference between therapeutic and toxic dosages. Digoxin levels in patients are often difficult to predict because of variation in the absorption of oral doses and the variation and non-renal excretion. Accordingly, the monitoring of serum digoxin levels is a valuable and necessary tool in decreasing patient toxicity risk and in detecting underdigitalization. This is particularly true since the incidence of toxicity increases from 5 to 71% for serum digoxin levels of 1.1 and 4.4 mg/mL, respectively.

Current digoxin immunoassay techniques include radioimmunoassay (RIA) systems, enzyme linked immunosorbent assays (ELISA) and EMIT assays. In radioimmunoassay systems, digoxin is generally labeled with radioactive iodine and the amount of labeled digoxin bound to an antibody is measured with a gamma-ray counting instrument. Such RIA systems present several drawbacks, such as the use of radioactive elements, sample instability, significant reagent preparation, expensive measuring instruments, etc.

ELISA and EMIT assays also require the labeling of digoxin, albeit with an enzyme, and the subsequent monitoring of an enzyme-substrate reaction. These assay systems, like the RIA assay, require significant reagent preparation, etc. Moreover, current assays systems typically include a serum pretreatment step in order to destroy interfering proteins which contribute to non-specific (i.e., serum interferant-digoxin antibody) reactions and lead to false positive results. For example, when performing an EMIT digoxin assay, the serum or plasma is mixed with sodium hydroxide to destroy interfering proteins.

### SUMMARY OF THE INVENTION

Accordingly, one object of this invention is to provide a novel immunoreactant-coated latex microparticle reagent for qualitative and quantitative immunoassays which is highly sensitive, specific and stable.

Another object of this invention is to provide a latex microparticle immunoassay reagent useful in the field of diagnostic testing, especially Toxic Drug Monitoring (TDM), and useful in latex agglutination inhibition assays.

Another object of this invention is to provide a latex-based microparticle reagent useful for the detection of cardiac glycosides and, in particular, for the detection of digoxin and digitoxin.

Another object of this invention is to provide a novel assay system which is sensitive, specific, stable and reliable and which overcomes the drawbacks of prior art assays.

Another object of this invention is provide a highly sensitive and specific digoxin assay system.

Another object of the present invention is to provide a highly sensitive and specific assay system and kit applicable to current commercial autoanalyzers useful in the field of clinical chemistry.

Another object of this invention is to provide a highly sensitive and specific cardiac glycoside assay system which can be operated without sample pre-treatment and which can be utilized to reliably determine the serum digoxin levels of patients simply and efficiently.

The above objects are achieved by a sensitized microparticle according to claim 1, an immunoassay method according to claim 11, a kit according to claim 12 and a further immonoassay method according to claim 18.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:

Figure 1 shows the calibration curves of latex microparticle reagents according to the present invention having coupled thereto a digoxin-human serum albumin (HSA) conjugate.

Figures 2a-d show a comparison between latex-based microparticle reagents according to the present invention sensitized with either a digoxin-HSA conjugate or a digoxin-bovine serum albumin (BSA) conjugate.

Figures 3a and b show the immunoassay results of latex microparticle reagents according to the present invention sensitized with a digoxin-HSA conjugate both with and without glycine after-treatment.

Figures 4a-d show the results obtained with a present invention digoxin assay system (Y axis) as a function of the results obtained with the Abbot TDx® method (X axis).

Figure 5 shows the long term stability of the present invention digoxin sensitized microparticle reagent and antibody buffer reagent.

Figures 6a and b show the results of a digoxin assay using the LPIA-100 system using 0.296 µm particles (Fig. 6a) and 0.434 µm particles (Fig. 6b).

Figures 7a and b show the effects of the concentration of sodium chloride on the invention buffer reagent in a present invention digoxin assay system.

Figures 8a and 8b show the effects of the concentration of choline chloride on a digoxin assay according to the present invention.

Figures 9a-d show the effect of fatty-acid-free human serum albumin (HSA) on a digoxin assay system accordng to the present invention.

### DESCRIPTION OF THE PREFERRED EMBODIMENTS

The latex-based microparticle reagent according to the present invention comprises any latex-based particle which is surface carboxylate-modified and which has covalently attached thereto an immunoreactant capable of specific reaction with a complementary immunoreactant. The latex-based particle of the present invention has an overall diameter of from 0.18-0.60 µm, preferably 0.18-0.5 µm, and a 0.08-0.35, preferably 0.10-0.30 nm² (8-35, preferably 10-30 square angstrom) carboxylate surface parking area. The term "surface parking area" as used herein refers to the surface area of the latex-based microparticle measured in nm² (square angstroms) divided by the total number of carboxylic acid (-COOH) and carboxylate (-COO⁻) functional groups on the surface thereof. "Carboxylate" refers to both COOH and COO⁻ functionalities. Additionally, the carboxylate-modified latex-based particles according to the invention preferably have densities ranging from 1.00 to 1.10, more preferably 1.02 to 1.06.

The latex-based particle of the present invention is any carboxylate-modified polymeric or copolymeric particle and may be made by any technique generally known in the art including emulsion polymerization, seeded emulsion polymerization and, preferably, suspension polymerization. The particles may be made with or without a crosslinking agent and include particles of a core-shell type. Any polymeric particle having sufficient surface carboxylate groups may be used in the present invention.

Particularly useful core particles include those made from C₁-C₈-, preferably C₁-C₂-(meth)acrylates which have been carboxylate-modified, carboxylate-modified polystyrene, and a mixture of acrylic acid and styrene monomers.

Methods for preparing carboxylate-modified latex microparticles useful in the present invention are known in the art and are described in, e.g., U.S. Patents 5,015,695, 4,988,770, 4,978,719 and 4,962 154.

As described above, the latex-based microparticle reagents of the present invention have a preferred diameter and a preferred carboxylate surface parking area. While the overall diameter of the invention particles may vary from 0.18-0.6 µm, preferred diameters are from 0.18-0.5 µm. Particularly useful diameters include 0.20, 0.25, 0.27, 0.30, 0.35, 0.37, 0.40, 0.45 and 0.47 µm. Further, it is preferred that the invention microparticle reagent be substantially spherical, preferably completely spherical, and it is preferred that a population of said microparticles be substantially uniform in size.

The invention microparticle reagents are carboxylate-modified and thus have free carboxylic acid and/or carboxylate moieties on the surface thereof depending upon the acidity of the medium they are or have been in contact with. The density of these acid and carboxylate residues is referred to as a surface parking area and is measured in nm² (square angstroms). Useful parking areas include 0.10, 0.12, 0.15, 0.18, 0.20, 0.22, 0.25, 0.28, 0.30 and 0.35 nm² (10, 12, 15, 18, 20, 22, 25, 28, 30, 32 and 35 square angstroms), and, in general, 0.08-0.35 nm² (8-35 square angstrom) parking areas give excellent results. The surface parking area of the invention microparticle reagents is calculated by the overall diameter and the number of acid groups. as determined by, e.g., titration, and the overall diameter of the invention microparticle reagents is measured by light scattering techniques, electron microscopy, etc.

The immunoreactant used to sensitize the invention latex microparticle reagent is any known immunoreactant capable of being covalently bonded to the surface carboxylate groups thereof. Mixtures of immunoreactants can be used, if desired. Included are hapten-carrier conjugates such as drug-protein conjugates, etc. Generally, the immunoreactants useful in the present invention are those capable of specific reaction with a complementary immunoreactant in any known immunoassay system including direct and competition assays, sandwich assays, etc.

In the present invention the term immunoreactant means any antigen or antibody optionally covalently, etc., attached to other molecules such as proteins or synthetic or natural polymers, etc., and the complementary immunoreactant means any antibody or antigen optionally covalently, etc., attached to other molecules such as synthetic or natural polymers, etc. capable of specifically binding to the immunoreactant. As used herein, the immunoreactant is the species covalently bound to the particle and the complementary immunoreactant is the specie that specifically binds thereto. Certain immunoassays, etc. may not require such an arrangement, however.

Examples of immunoreactants and complementary immunoreactants useful in the present invention include the following:

| | |
|---|---|
| AFP | Alpha-fetoprotein |
| Beta-2-microglogulin | |
| CEA | Carcinoembryonic antigen |
| Ferritin | |
| CA 19-9 | Carbohydrate antigen 19-9 |
| PAP | Prostatic acid phosphatase |
| PSA | Prostate-specific antigen |
| CRP | C-reactive protein |
| Mb | Myoglobin |
| RF | Rheumatoid factor |
| ASO | Anti-streptolysin-O |
| FDP | Fibrin degradation product |
| Anti-thrombin-III | |
| Plasminogen | |
| Alpha-2-plasmin inhibitor | |
| D-dimer | Fibrin degradation product D-fragment dimer |
| IgG | Immunoglobulin G |
| IgA | Immunoglobulin A |
| IgM | Immunoglobulin M |
| IgE | Immunoglobulin E |
| C3 | Complement 3 |
| C4 | Complement 4 |
| Urinary albumin | |
| hCG | human chorionic gonadotropin |
| hPL | Human placental lactogen |
| Insulin | |
| HBs antigen | Hepatitis-B surface antigen |
| HBs antibody | Anti-hepatitis-B core antigen |
| | antibodies |
| HBc antibody | Anti-hepatitis-B core antigen |
| | antibodies |
| HCV antibody | Anti-hepatitis-C virus |
| | antibodies |
| Treponema | Anti-treponema pallidum |
| | antibodies |
| TSH | Thyroid stimulating hormone |
| LH | Lutenizing hormone |
| FSH | Follicle stimulating hormone |
| Digoxin | |
| Digitoxin | |
| Quinidine | |
| Procainamide | |
| NAPA | N-acetyl procainamide |
| Theophylline | |
| Phenytoin | |
| Phenobarbital | |
| Carbanazepine | |
| Valproic acid | |
| Ethosuccimide | |
| Gentamicin | |
| Tobramycin | |
| Amikacin | |
| Vancomycin | |
| Cyclosporin-A | |
| B12 | Vitamin B12 |
| Folic acid | |
| T3 | Triiodothyronine |
| T4 | Thyroxine |
| Estrogen | |

All of the above-identified species can be called either an immunoreactant or a complementary immunoreactant depending on their role in a given immunoassay. Pairing is important, the name is not, and where only an immunoreactant is referred to herein any of the above species and similar known species are meant. Naturally, all immunoreactants complimentary to those listed above are included in the present invention immunoreactants. Other immunoreactants useful in the present invention are described in the Pierce Immunotechnology Catalog and handbook, Pierce Chemical Co., 1992. As mentioned above, protein conjugates such as HSA and BSA conjugates of haptens are included in the invention immunoreactants. Specific examples of such carrier-hapten conjugates include digoxin-HSA and digoxin-BSA. Methods for producing these conjugates are explained in Erlanger, B. Meth. of Enzym 70, 85, 1980 and Bauminger et al., Meth. in Enzym., 70, 151, 1980.

In the present invention the immunoreactant is covalently bound to the carboxylate-modified microparticle. Once the immunoreactant is bound to the microparticle, the microparticle is described as being sensitized.

The immunoreactant-coated microparticle reagent (sensitized microparticle reagent) of the present invention is prepared by covalently bonding the immunoreactant to the carboxylate-modified latex microparticle. Any method known in the art for covalently attaching immunoreactants to carboxylate groups may be used. Several techniques for the immobilization of immunoreactants on solid supports useful in the present invention are described in Immobilized Affinity Ligand Techniques, by G. T. Hermanson, et al., 1992, in U.S. patents 4,045,384, 4,140,662 and 4,680,338, in Quasla, G. et al., J. Immuno. Meth., Vol. 22, 165, 1978, Srere, P. et al., Meth. Enzym., 44, 11, 1976, Nustad, K. et al., Devel. Biol. Stds. 57, 321, 1984, Bahadur, A. et al, Makromol. Chem., 186, 1387, 1985, Margel, S. et al., J. Immuno. Meth. 28, 341 1979, and Suen, C. et al., Makromol. Chem. 186, 255, 1985. A linker molecule may be used.

A preferred method for covalently bonding the invention immunoreactant to the invention latex-based particle is through the use of a carbodiimide coupling reagent. The use of carbodiimide coupling reagents to effect the condensation of a carboxylic acid functionality with an amine is well-known in the art and described in Organic Chemistry by Streitweiser and Heathcock, MacMillan, 1976, by Sheehan, J. C., et al., in J. Amer. Chem. Soc. 95, 875 (1973), by Thomas, J. O., et al., in J. Mol. Biol., 123, 149 (1978), by Packer, L., et al., in FEBS LETT., 108, 243 (1979), in Anal. Biochem., 63, 485 (1975), in Plant Physiol., 53, 619 (1974), and in J. Org. Chem., 21, 439 (1956),

Particularly preferred carbodiimide coupling reagents for condensing the surface carboxylate groups to the immunoreactant include dicyclohexylcarbodiimide (DCC), 1-ethyl-3-(3-dimethylaminopropyl)-carbodiimide (EDC) and 1-cyclohexyl-3-(2-morpholinoethyl) carbodiimide (CMC). EDC is particularly preferred due to its high solubility, effectiveness under mild conditions and ease of reaction control. The amount of immunoreactant covalently bound to the invention particle varies depending on the particle characteristics and immunoreactant. In general, 50-10,000, preferably 200-2000 immunoreactant molecules are bound to a typical invention microparticle. However, and depending on the assay technique, desired rate of reaction, etc., this range can be altered.

Typically, the amount of carbodiimide used to effect coupling is from 0.01 to 5 percent of the weight of the particles, preferably 0.05 to 2 percent, or 2-5 times the equivalence of the latex surface carboxylate groups. See U.S. 4,181,636 and 4,045,384.

Carbodiimide-catalyzed coupling of carboxyl groups to amino groups can produce undesirable by-products which can provide reaction sites for non-specific reactions. In order to counter the formation of such by-products, an excess amount of an amine can be added after the coupling reaction has progressed sufficiently, and any carbodiimide-modified surface carboxylate residue can be returned to an acid or a carboxylate functionality through the use of an amino acid. In this manner, the surface charge of the invention microparticle reagent remains relatively constant and controllable. Preferred amines useful for quenching the carbodiimide coupling reaction include ethanolamine, ethylenediamine, glucosamine, glycine and lysine. Glycine and lysine are preferred, and glycine is particularly preferred.

Prior to effecting coupling of the immunoreactant to the particle it is preferred to clean the microparticle by, e.g., passing the particle through an ion exchange resin such as Bio-Rad mixed bed resin AG 501-X8.

When an invention latex-based microparticle reagent is provided according to the present invention, high sensitivity and good dispersibility are provided. In particular, the combination of adequate particle size and relatively small surface carboxylate parking area provide a reagent particle sensitive enough to quantitate an analyte present in an amount less than 1 ng/ml. This range of sensitivity is essential for several drug monitoring applications, including digoxin assays. Further, the sensitized reagent microparticles of the present invention have excellent dispersibility in aqueous solvents and thus provide a homogeneous reagent for particle-based immunoassays yielding both reproducible results and excellent storage stability. The particular surface carboxylate parking area, particle size, etc., of the present invention reagent all make the invention reagent less susceptible to the serum matrix effect and thus improves the specificity of the reagent.

Obviously, numerous modifications and variations of the present invention are possible in light of the above teachings. It is therefore to be understood that within the scope of the appended claims, the invention may be practiced otherwise than as specifically described herein.

Furthermore, the present invention is directed to an immunoassay system and immunoassay reagents capable of determining the presence and amount of a desired species in a sample. These reagents include the above-described latex-based microparticle, and a buffer reagent comprising buffering components, sodium chloride, choline chloride, fatty-acid-free serum albumin, a polysaccharide like sodium dextransulfate or methyl cellulose, optionally a complimentary immunoreactant and, optionally, a secondary or tertiary amine non-specific reaction suppressor. Kits with two container means, one containing the above-described latex-based microparticle and the other containing the buffer components, are also provided. Any container means can be used, including vials, jars, tubes, bottles, foil packs, etc. Removably-sealed and unsealed containers can be used. The container means and sealing means can be made from any material such as glass, plastic metal, composite, etc.

The invention reagents can be used in any immunoassay system or method which utilizes the above-described latex-based microparticle which has been sensitized with an immunoreactant and a buffer reagent. Several immunoassay methods and automated and semi-automated devices in which the present invention reagents may be used are described and explained in the Handbook of Experimental Immunology, vols. 1-4, Blackwell Scientific 1987, in U.S. Patents 3,088,875, 3,857,931, 3,992,517, 4,080,264, 4,174,952, 4,203,724, 4,480,042, 4,590,156, 4,690,906, 4,716,123, 4,772,550, 4,851,329, 4,960,692 and 5,100,805, and in Grange, J. et al., J. Immuno. Meth., 18,365,1977, Hechemy, K. et al., Lab Management, 27 June/July 1984, Looney, C., J. Clin. Immunoassay, 7,(1), 90, 1984, Von Schulthes, G. et al., Mol Immunol, 1, 81, 1980, Craine, J., Am. Biotech, Lab., 34, May-June, 1987, Heveran, J., J. Forensic Sci., 470, 1977 and Kimura, H. J. Immuno. Meth., 38, 353, 1980.

Competitive and noncompetitive methods are included.

Preferably, a particle-based immunoassay system is utilized with the present invention immunoassay reagents, and immunoassay systems based upon latex agglutination or latex agglutination inhibition are most preferred (see U.S. 4,203,724 and 5,100,805, and J. Clin. Chem. 38(b), 1012 (1992)). Analyzers useful for conducting immunoassays using the present invention reagents and methods include the LPIA-100 fully automated latex immunoassay system of Mitsubishi Kasei Corporation, Japan, the COBAS FARA and COBAS MIRA sytems of Roche Diagnostic Systems, Inc. and the Hitachi 704 analyzer.

When using latex particle agglutination or agglutination inhibition methods, any quantitative photometric instrument which has the capability of dispensing the sample and, optionally, an antibody reagent, into a cuvette to allow preincubation followed by the dispensing of a sensitized solid reagent into the cuvette and monitoring the ensuing reaction photometrically is preferred. Of course, any instrument capable of measuring agglutination or agglutination inhibition by, e.g., light scattering technicques, etc. can be used and sample addition, reagent addition, etc. can be conducted by hand or by semi-automated methods.

The buffer reagent of the present invention comprises buffering agents, sodium chloride, choline chloride, a polysaccharide compound such as one or both of sodium dextransulfate and methyl cellulose, optionally a complimentary immunoreactant which specifically reacts with the immunoreactant on the invention sensitized support, fatty-acid-free serum albumin and, optionally, a secondary or tertiary amine non-specific reaction suppressor.

The invention antibody buffer reagent is preferably water-based and has a pH of from 4.5 to 10, preferably 5.5 to 9.5. The amount of sodium chloride present in the buffer may vary from 1.0 to 5, preferably 1.5 to 4.5, most preferably 2.0-4.0 wt%. The amount of choline chloride present in the invention reagent buffer is from 1 to 15%, preferably 2-12%, most preferably 4-8% by weight. Typical pH buffering agents (the term "a buffering agent" means those single substances or combination of substances which resist a change in hydrogen ion concentration upon the addition of acid or alkali) are used in the present invention antibody buffer reagent. Examples include Tris-(hydroxymethyl)-aminomethane, phosphate buffering agents, those listed in the Pierce Immunotechnology Catalogue and Handbook, etc. The weight percentages above and below are based on the total buffer weight.

The present invention buffer reagent also includes at least one polysaccharide like sodium dextransulfate, methyl cellulose, etc. Other examples incluede carboxymethylcellulose, dextran, etc. The polysaccharide thickens the buffer reagent to an acceptable viscosity. Typically, the amount of sodium dextran sulfate useful in the present invention buffer reagent is from 0.2-3.0, preferably 0.6-2.0, most preferably 1.0-1.8 weight percent. The amount of methyl cellulose useful in the present invention buffer reagent is from 0.05-1.0, preferably 0.1-0.4, most preferably 0.15-0.3% weight percent.

The fatty-acid-free serum albumin useful in the present invention is characterized in that it is substantially free of fatty acids. The material can be prepared by the method of Chen, R.J., J. Biol. Chem., 242,173 (1967), and may be commercially obtained in varying species and grades from Sigma and Miles. Human, bovine, rabbit, sheep, etc. serum albumins can be used. The amount of fatty-acid-free serum albumin useful in the present invention buffer is from 5-100, preferably 7.5-60, most preferably 10-40 mg/mℓ of buffer. Fatty acid free human serum albumin is most preferred. Materials that are fatty acid free and globulin free are also preferred.

The optional complimentary immunoreactant useful in the present invention buffer reagent is any complimentary immunoreactant which specifically reacts with the immunoreactant used to sensitized the above-described latex-based microparticle. This species is optional since, e.g., in direct immunoassay in which the species in the sample being detected or quantitated reacts directly with the latex-based microparticle no complimentary immunoreactant in solution is necessary. When used, preferred complimentary immunoreactants are monoclonal antibodies which specifically react with the immunoreactant used to sensitized the microparticle. Anti-digoxin monoclonal antibodies are particularly preferred. Methods for producing monoclonal antibodies are well known in the art and are described in D.E. Yelton et al., Annu. Rev. Biochem., 50, 657, 1981, Milstein, C., Sci. American, 243(4), 66, 1980; Kennett, R.H. et al. Monoclonal Antibodies, Plenum Press, New York, 1980 and Kohler, G. et al., Nature, 256, 495 (1975).

As described above, an optional component of the invention buffer reagent is one or more of a secondary or tertiary amine which improves the accuracy and reliability of the invention immunoassay reagents and methods by significantly reducing or eliminating non-specific interactions.

The secondary and tertiary amine non-sepcific reaction suppressors useful in the present invention are those of the formula: 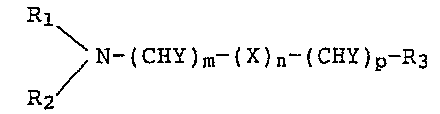 wherein
X is -NH-(CO)-NH-, -NH-(CS)-NH-, 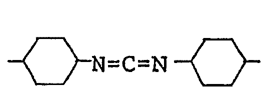 or -N=C=N-, R₁ and R₂, which may be the same or different, are C₁-C₅ linear or branched alkyl groups, or R₁ and R₂, together with nitrogen, is 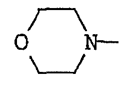 or the metho-p-toluenesulfonate salt thereof,
Y, which may be the same or different, is any of H, OH and halogen (Br, Cℓ, F),
R₃ is -NR₁R₂, -NH₂, -CHY, cyclohexyl, or H,
m is an integer of from 0 to 5,
p is an integer of from 0 to 5, and
n is 0 or 1,
provided that at least one of m and p is at least 1 when n equals 1, and provided that when m=n=p=0, R₃ is H or -CH₂Y, and the acid addition salts thereof, particularly the HCℓ salts, phosphoric acid salts and sulfuric acid salts thereof. A preferred group of suppressors are those where m is at least 1 when n=1.

These secondary and tertiary amines may be substituted on any or all of the m and p methylenes with any combination of H, OH and halogen, include compounds where m=n=p=0 and R₃ is, e.g., H or methyl, compounds where m, optionally n and optionally p are not 0 and R₃ is H or -CH₂Y, etc.

Several of the above-described compounds are the hydrolysis products of carbodiimides useful in the preparation of peptides. See Sheehan, J.C., et al., J. Org. Chem., 26, 2525, 1961 and Staros, J.V., et al., Anal. Biochem., 156, 220, 1986.

One particularly preferred non-specific reaction suppressor is 1-ethyl-3-(3-dimethylaminopropyl)urea (EDU), i.e., a compound according to the formula above where R₁=R₂=methyl, Y=H, m=3, n=1, p=1 and R₃=methyl. Another is 1-cyclohexyl-3-(2-morpholinoethyl) carbodiimide metho-p-toluenesulfonate (CMC). These compounds and their hydrolysis products are useful for suppressing non-specific reactions in immunoassay, particularly immunoassay wherein an immunoreactant is attached covalently or by adsorption to a microparticle.

The invention non-specific reaction suppressors are generally commercially available and prepared by simple organic reactions well known to those of ordinary skill in this art and explained in, e.g., Introduction to Organic Chemistry, A. Streitwieser and C. Heathcock, Macmillan, 1976; Reagents for Organic Synthesis, Feiser and Fieser, John Wiley and Sons, 1967 and succeeding volumes; Survey of Organic Syntheses, John Wiley and Sons, Vols I and II, 1970; and Advanced Organic Chemistry, March, Wiley, 1985. For example, the urea compounds (-NH-CO-NH-) can be prepared by hydrolysis of the carbodiminde (-N=C=N-) compounds.

The non-specific reaction suppressor of the present invention described by the above formula may be used singly or in combination and may be added to the invention buffer reagent or adsorbed on the above-described latex-based microparticle before or after sensitization. Further, the non-specific reaction suppressor can be utilized in combination with conventional non-specific reaction suppressors.

A particularly preferred embodiment of the present invention is one where a compound according to the above formula is used to suppress non-specific immunoassay reactions in a system in which an immunoreactant has first been bound to a solid substrate using a carbodiimide reagent such as 1-ethyl-3-(3-dimethylaminopropyl)-carbodiimide hydrochloride (EDC) or CMC. A further preferred embodiment of the present invention is one where the non-specific reaction suppressor utilized is the hydrolysis product of the carbodiimide used in the binding of the immunoreactant to the microparticle.

Wide ranges of suppressor concentration are effective. In general, the amount of non-specific reaction supressor useful for suppressing non-specific immunoassay reactions according to the invention is from 0.1 to 300 mM, preferably 0.5 to 50 mM, more preferably 1.25 to 25 mM based on the total immunoassay solution volume, the total buffer volume, the solution volume of a sample to be tested, or on the solution volume of a microparticle sensitized with immunoreactant suspended in a solvent.

The sensitized microparticle and buffer reagents of the present invention allow for highly sensitive and specific immunoassay determinations. When used in a direct-measurement (i.e., non-inhibition mode) assay the complimentary immunoreactant is omitted from the invention buffer composition, and only sodium chloride, choline chloride, polysaccharide, fatty-acid-free serum albumin and, optionally, one or more of the secondary or tertiary non-specific reaction suppressors are used with conventional buffering ingredients. When an assay based on inhibition is used a complimentary immunoreactant is present in the invention buffer reagent and the sample to be tested is contacted with the buffer reagent and incubated for from 1-20 minutes. Upon completion of incubation the sensitized microparticle is added to the mixture to bind unoccupied complimentary immunoreactant binding sights, and the rate of binding is inversely related to the immunoreactant concentration in the serum sample.

The present invention immunoassay reagents, immunoassay systems, and methods comprising them provide highly sensitive and specific determinations of specie concentration in samples, particularly patient serum samples. For example, an immunoassay system based upon latex agglutination inhibition and utilizing the preferred invention latex-based digoxin-HSA sensitized microparticle reagent and an antibody buffer reagent comprising sodium chloride, choline chloride, sodium dextransulfate or methyl cellulose, an anti-digoxin monoclonal antibody, fatty-acid-free serum albumin and e.g., 1-ethyl-3-(3-dimethyaminopropyl)-urea provides a precise and reliable assay system for determining the amount of digoxin in patient sera. This is particularly true for all the invention reagents and for the preferred reagents when the volume of the serum or plasma specimen used is less than 4% of the total immunoassay system volume, and can be accomplished without any special sample pre-treatment such as deproteination, resin treatment, ultra filtration, etc.

When the reagents of the present invention are utilized with automated, e.g., photometric, instruments commercially available and having the capability of dispensing both sample and buffer (optionally containing a complimentary immunoreactant) into a cuvette and dispensing a solid sensitized reagent into the cuvette with mixing one can quantitate the amount of sera immunoreactant for large numbers of patient samples in a short amount of time with excellent reliability and sensitivity.

The superior results afforded by the present invention reagents and immunoassay systems and methods using them (both general and preferred embodiments) are derived from several aspects of the above-described invention including the use of the fatty-acid-free serum albumin in the buffer reagent (and/or pre-applied on the sensitized microparticle if desired) and the optional use of the secondary or tertiary non-specific reaction suppressors in the buffer reagent (and/or absorbed on the sensitized microparticle, if desired). Due to the sensitivity of the present reagents and methods using them, small amounts of sample may be used while maintaining accuracy and reliability. Conventional immunoassay reagents and methods sacrifice sensitivity when sample volume is reduced: the present invention reagents and immunoassay using them maintain high sensitivity even at low sample volume.

The following examples further describe the present invention, but the invention is not limited thereto.

### EXAMPLES

### Preparation of Latex-based Microparticle

A 10 gallon glass lined reactor fitted with a condenser and stirrer was charged with 83 gms of sodium bicarbonate, 14,000 gms of deionized water and a surfactant (224 gms of MA-80). This mixture was heated to 71°C (160°F) and purged with argon for 10 minutes. 208 gms of acrylic acid and 11,200 gms of styrene were mixed together and purged with argon and charged on top of the aqueous phase. The resulting emulsion was allowed to equilibrate 10 minutes.

33.6 gms of potassium persulfate was dissolved in 2000 gms deionized water, purged with argon and charged to the glass lined reactor to initiate polymerization. After 8 hours the reactor was cooled and discharged to obtain 28,000 gms of a 40% by weight suspension of 0.138 µm carboxylate-modified microparticles. Conduct iometric titration showed 0.931 milliequivalents per gram of weak acid.

### Preparation of Digoxin Reagents

### 1. Conjugate preparation

A digoxin-HSA conjugate was prepared according to a modified method of Smith, T. W., et al., Biochemistry, 9, 331 (1970) and Bulter, U.P. et al, Proc. Natl. Acad. Sci. U.S., 57, 71 1967, A representative protocol is as follows:

To 0.5 g of digoxin (obtained from Sigma) suspended in 20 ml of absolute ethanol at room temperature was added 20 ml of 0.1 M sodium metaperiodate dropwise with stirring. After 25 minutes, 0.6 ml of 1 M ethylene glycol was added. Five minutes later, the reaction mixture was added dropwise with stirring to 0.6 g of human serum albumin (obtained from Sigma) in 20 ml of 9.5 pH water (adjusted with K₂CO₃). The pH of the aqueous solution was maintained in the range of from 9.0-9.5 by the dropwise addition of 5% K₂CO₃. After 45 minutes, the pH was stable and 0.3 g of sodium borohydride freshly dissolved in 20 ml of water was added thereto. Three hours later, 7.6 ml of 1 M formic acid was added to lower the pH to 6.5 and, after 1 hour, the Ph was raised to 8.5 by the addition of NH₄OH. The entire reaction mixture was then dialyzed against cold running tap water for 4 days and finally dialyzed against 0.1M NaHCO₃ with 0.05% NaN₃. The solution was kept in a refrigerator.

### 2. Sensitized microparticle reagent preparation

### A) Ion exchange treatment of latex particle:

To 100 ml of a carboxylate-modified latex particle suspension [10% solids; 0.292 µm diameter; 0.31 meq carboxylate/g, 0.105 nm² (10.5 square angstrom) parking area, particle made by Seradyn, Inc.] is added 20 g of a mixed-bed ion exchange resin (BioRad AG 501-X8) with slow stirring for 2 hours at room temperature. The suspension is then filtered using glass fiber filters to remove the resin, and the latex is ready for coupling.

### B) Latex particle coupling reaction for digoxin-HSA conjugate:

To a 50 ml polycarbonate centrifuge tube is added 15 ml of 0.1 M bicarbonate buffer, pH 8.0, and 5 ml of the above cleaned 10% solids latex suspension, and then incubated at 37°C for 10 min with stirring prior to reaction. 5 ml of 88 mg/ml 1-ethyl-3-(3-dimethylaminopropyl)-carbodiimide hydrochloride (EDC; freshly dissolved with water) is added to the mixture to activate carboxyl residues on latex surface for 10 min. After activation, 2.5 ml of 10 mg/ml of the above digoxin-HSA conjugate is added with vigorous stirring and incubated for 10 min. The reaction is stopped by adding 5 ml of 500 mM glycine buffer, pH 8.5. Another 10 min incubation is done to ensure complete termination.

### C) Washing of the conjugate coupled (i.e., sensitized) latex reagent:

The digoxin-HSA conjugate coupled latex particles are centrifuged at 26,000 x g for 20 min. The supernatant is discarded, and to the pellet is added 25 ml of water. The pellet is then resuspended by vigorous stirring and washing is repeated four times; however, in the last resuspension, 0.05% sodium azide solution is used as storage medium. Finally, the latex suspension is sonicated and diluted to the concentration (normally 0.1-0.4% solids) ready for use.

### 3. Antibody reagent composition (EXAMPLE 1 ∼ 4) :

An antibody reagent is prepared by dissolving the following materials in water and adjusting the pH with hydrochloric acid. Preferably, the antibody is added last.
3.7% NaCl
200 mM Tris-(hydroxymethyl)-aminomethane, pH 8.0
1.2% Sodium Dextransulfate
1 mg/ml HSA
0.05% Sodium azide
1:60,000 diluted anti-digoxin mouse monoclonal antibody (obtained from Beekman).

### 4. Preparation of Anti-digoxin Antibody Buffer Reagent Composition (EXAMPLE 5 ∼ 10)

A digoxin antibody reagent was prepared by dissolving the following materials in water and adjusting the pH with hydrochloric acid. The antibody was added last.

| | |
|---|---|
| 4.375% | NaCl |
| 250 mM | Bis-Tris, pH 6.5 |
| 6.4% | Choline Chloride |
| 1.25-1.75% | Sodium Dextransulfate |
| 2.0% | Fatty-acid-free human serum albumin (FAF-HSA) |
| 25 mM | 1-Ethyl-3-(3-dimethylaminopropyl)-urea |
| 0.1% | Sodium azide |
| 0.01% | Antifoam 1410 (Dow Corning) |
| 1:86,000-120,000 | Diluted anti-digoxin monoclonal antibody |

### Digoxin inhibition assay conditions and protocol (EXAMPLE 1 ∼ 4)

### 1. Instrument:

The LPIA-100® (Mitsubishi Kasei Corporation) was used for the assay.

### 2. Assay conditions:

10 µl Specimen
50 µl Pushing buffer, 0.9% NaCl, 0.05% NaN₃
200 µl Antibody reagent
40 µl Microparticle reagent
650 or 950 nm Wavelength

### 3. Assay procedure:

The digoxin assay used was based on the inhibition of the agglutination reaction between antibody and antigen-coated latex particle. A serum sample containing digoxin is incubated for ten-minutes at 37°C with the anti-digoxin antibody diluted in assay buffer. Upon completion of incubation, the digoxin-coated microparticle reagent is added to bind unoccupied antibody binding sites, and the rate of agglutination is measured by the increase in absorbance at 650 or 950 nm for 10 min. The rate of increase in absorbance is inversely related to the digoxin concentration in the serum sample.

| Assay Parameters for chemistry analyzers (EXAMPLE 5 ∼ 10) | | | | |
|---|---|---|---|---|
| Instrument name | COBAS FARA | COBAS MIRA | HITACHI704 | LPIA100 |
| Wavelength | 750 nm | 600 nm | 700 nm | 650 or 950 nm |
| Temperature | 37°C | 37°C | 37°C | 37°C |
| Sample volume | 6 µl | 6 µl | 15 µl | 10 µl |
| Antibody reagent | 120 µl | 120 µl | 220 µl | 200 µl |
| Latex reagent | 25 µl | 20 µl | 160 µl (3x diluted) | 40 µl |
| Pushing water/Buffer | 20 µl | 35 µl | - | 50 µl |
| Sample/Antibody Preincubation | 10 min | 7 min | 5 min | 10 min |
| Reading time | 5 min | 4 min | 5 min | 10 min |

### EXAMPLE 1

### 1. Calibration curves of digoxin reagents

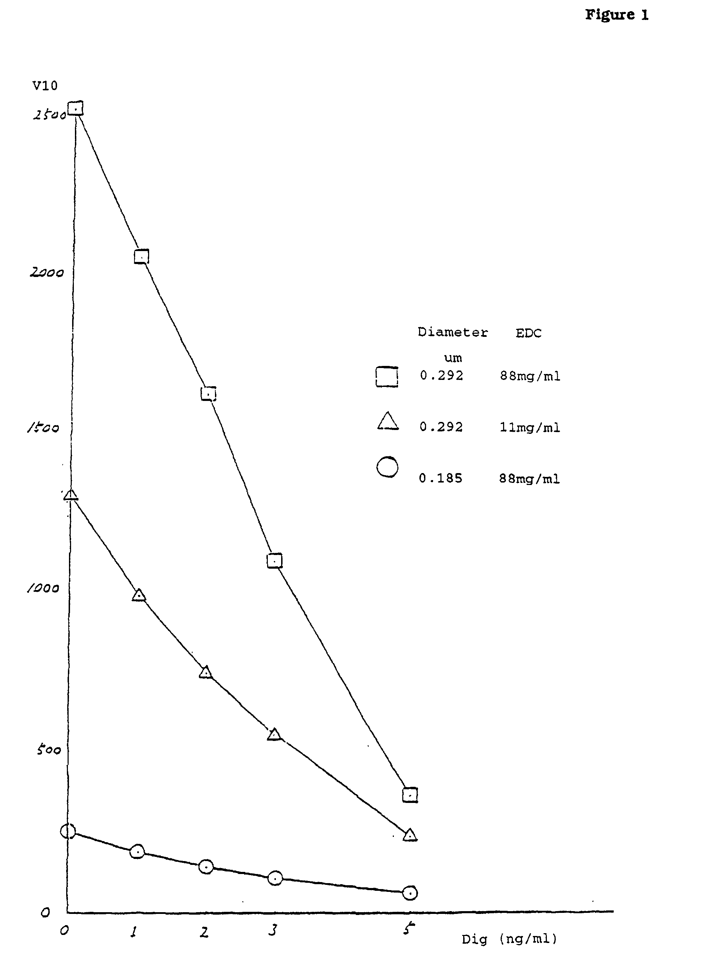

In addition to the 0.292 µm diameter particle reagent described above, another particle reagent was made with the same particle, but using a lower concentration of EDC (11 mg/ml), and a reagent with a diameter of 0.184 µm was prepared according to the above protocol using 88ng/ml of EDC, and all three were evaluated by measuring their digoxin calibration curves. Figure 1 shows these calibration curves made with samples having known concentrations of digoxin while measuring the rate of increase in absorbance at 650 nm with the LPIA-100 system. The curves indicate that reagents having a larger particle size have higher sensitivity (the absolute values of the rates are higher), and that all the calibration curves are satisfactory considering the therapeutic range of digoxin (0.5-2.0 ng/ml).

### 2. Precision of 0.184 µm diameter particle reagent

According to Figure 1, the 0.184 µm sensitized particle reagent is the least sensitive. However, the reagent still has excellent performance, and provides excellent precision as is shown in Table 1.

**Table 1.**

| Precision study using 0.184 µm particle reagent. | | | | | | |
|---|---|---|---|---|---|---|
| Digoxin Calibrator Concentration (ng/ml) | | | | | | |
| n=10 | 0.0 | 0.5 | 1.0 | 2.0 | 3.0 | .5.0 |
| Rate (mA/min) | | | | | | |
| mean | 257.4 | 228.0 | 200.1 | 148.9 | 116.0 | 66.0 |
| S.D. | 4.3 | 3.8 | 4.0 | 4.3 | 3.6 | 4.3 |
| C.V.% | 1.7 | 1.7 | 2.0 | 2.9 | 3.1 | 6.5 |
| Concentration calibrated | | | | | | |
| mean | 0.12 | 0.56 | 0.98 | 2.13 | 3.10 | 5.43 |
| S.D. | 0.06 | 0.06 | 0.07 | 0.12 | 0.15 | 0.20 |
| C.V.% | - | 10.0 | 7.5 | 5.6 | 4.8 | 3.7 |

where n is the number of samples tested, S.D. is the standard deviation and C.V.% is the coefficient of variation. The concentration calibrated refers to digoxin calibrator set (Roche Diagnostic Systems, Inc.).

### EXAMPLE 2

Fourteen different sensitized carboxylate-modified latex particles were compared in terms of their dispersibility and reactivity in the above-described digoxin inhibition assay. Microparticle reagent preparation was done based on the same preparation protocol as EXAMPLE 1. Reactivity was measured using 0 ng/ml serum calibrator as the sample.

Table 2 shows the results obtained which demonstrate the importance of adequate parking area in providing reactive and dispersible microparticle reagents.

**Table 2.**

| Evaluation of 14 different sensitized microparticle digoxin reagents. | | | | | |
|---|---|---|---|---|---|
| Sample | Diameter (µm) | Parking area (2) | Dispersibility during: | | Reactivity (mA/min) Rate |
| | | | Conjugation | Assay | |
| At 650 nm: Antibody 1:400,000: | | | | | |
| 1 | 0.184 | 18.8 | Excellent | Excellent | 259.6 |
| 2 | 0.203 | 51.9 | Aggregated | Aggregated | 62.1 |
| 3 | 0.210 | 56.8 | Clumped | Clumped | 0.2 |
| 4 | 0.215 | 150.6 | Clumped | Clumped | -0.4 |
| 5 | 0.264 | 34.2 | Fair | Aggregated | 61.1 |
| 6 | 0.283 | 119.7 | Aggregated | Aggregated | 78.6 |
| 7 | 0.284 | 16.1 | Excellent | Good | 1319.0 |
| 8 | 0.285 | 69.5 | Fair | Aggregated | 70.1 |
| 9 | 0.292 | 10.5 | Excellent | Excellent | 1104.2 |
| 10 | 0.296 | 12.0 | Excellent | Excellent | 1314.4 |

| At 950 nm; Antibody 1:120,000: | | | | | |
|---|---|---|---|---|---|
| 9 | 0.292 | 10.5 | Excellent | Excellent | 248.3 |
| 11 | 0.400 | 58.8 | Aggregated | Aggregated | 78.4 |
| 12 | 0.406 | 4.02 | Excellent | Excellent | 79.4 |
| 13 | 0.434 | 25.9 | Fair | Good | 763.4 |
| 14 | 0.490 | 2.56 | Excellent | Excellent | 41.8 |

| | | | | | |
|---|---|---|---|---|---|
| (2) 1 square angstrom ≙ 0.01 nm² | | | | | |

### EXAMPLE 3

Two digoxin conjugates were prepared with bovine serum albumin (BSA) according to Smith, supra, and human serum albumin (HSA) as above. Particles were sensitized as above and were compared using the LPIA system and 50 serum specimens pre-evaluated by TDx® (Abbot). The sensitized latex particles were prepared using 0.184 µm particles according to the protocol above, and two different lots of anti-digoxin monoclonal antibody were used to make sure the results were due to the sensitized particles and not the particular antibody batch.

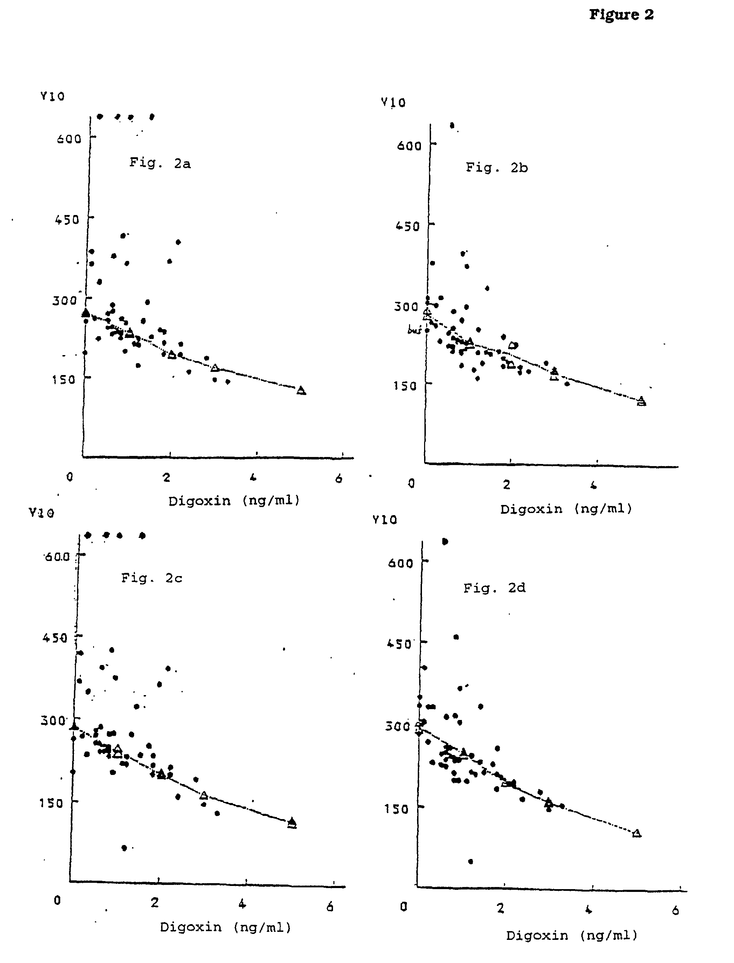

Figures 2a and 2c show the results obtained with the latex reagents sensitized with the digoxin-BSA conjugate (black dots), the results in 2a and 2c using different antibody lots, and Figures 2b and 2d show the results of particles sensitized with digoxin-HSA using different lots of antibody (black dots).

The triangles in all Figures 2a-2d are results obtained by the LPIA-100 system using digoxin calibrators.

As is seen in Figures 2a, b, c, d, the difference of antibody lot had a minimal (if any) effect on the tests, and the digoxin-HSA sensitized microparticles provided better data distribution than did digoxin-BSA sensitized particles.

### EXAMPLE 4

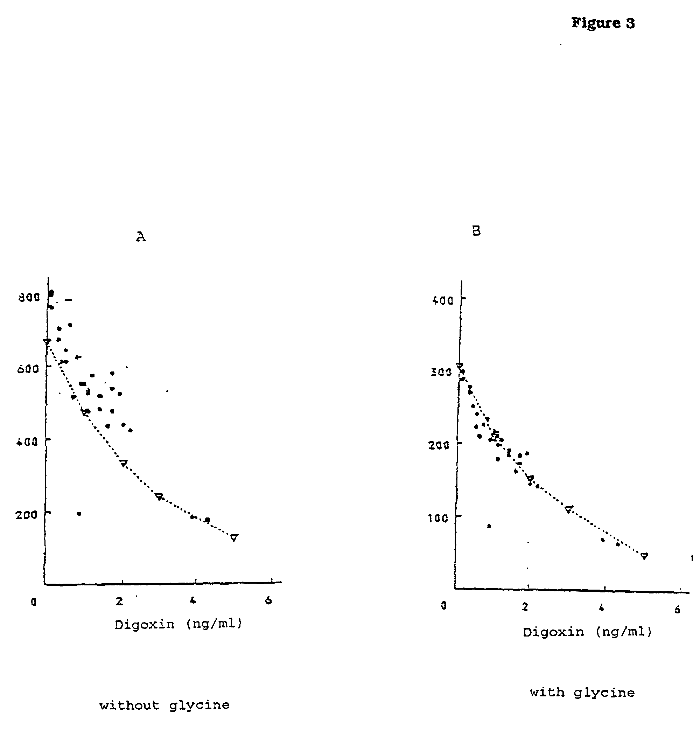

The digoxin assay described above was performed using a 0.184 µm particle sensitized with digoxin-HSA prepared with and without glycine treatment after carbodiimide condensation reaction. In the assay, five calibrators were measured in the LPIA-100 system and 35 serum specimens were pre-evaluated by TDx® method were then tested in the LPIA-100 instrument. The results are shown as data distribution graphs together with the calibration curves in Figures 3a (without glycine after-treatment) and 3b (with glycine after-treatment).

As is shown in Figures 3a and 3b, glycine treatment reduced reactivity somewhat (i.e. lower rate values), but made sample distribution much tighter, thus improving reagent accuracy.

### EXAMPLE 5

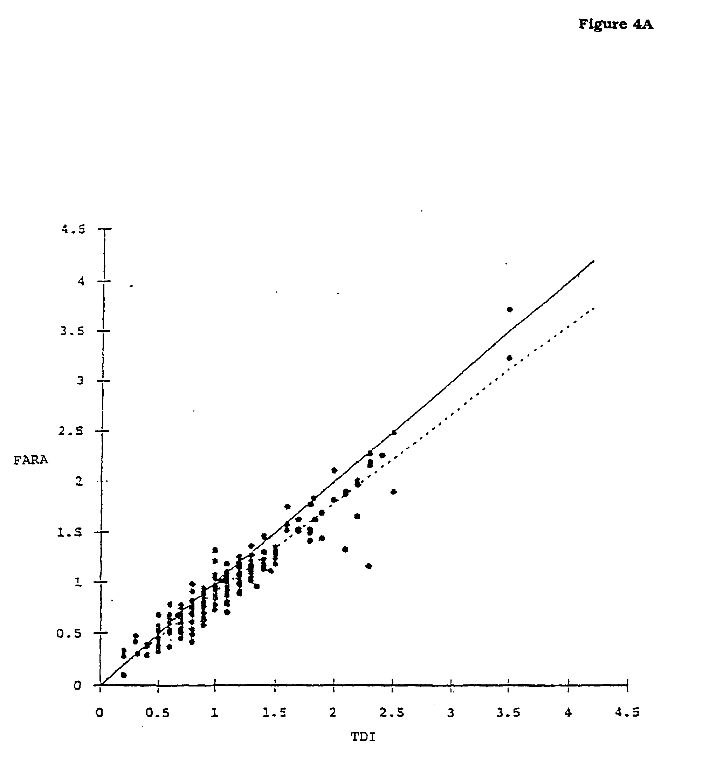

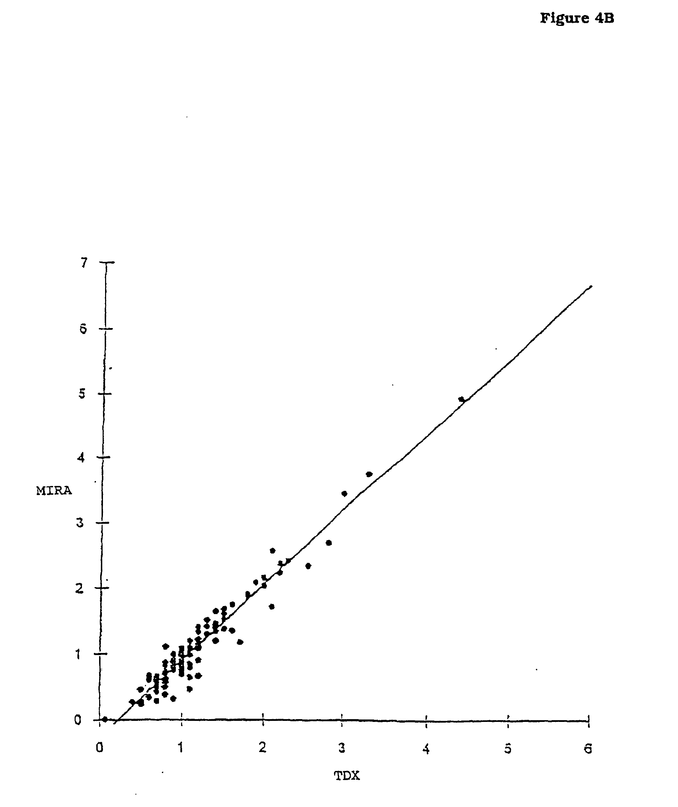

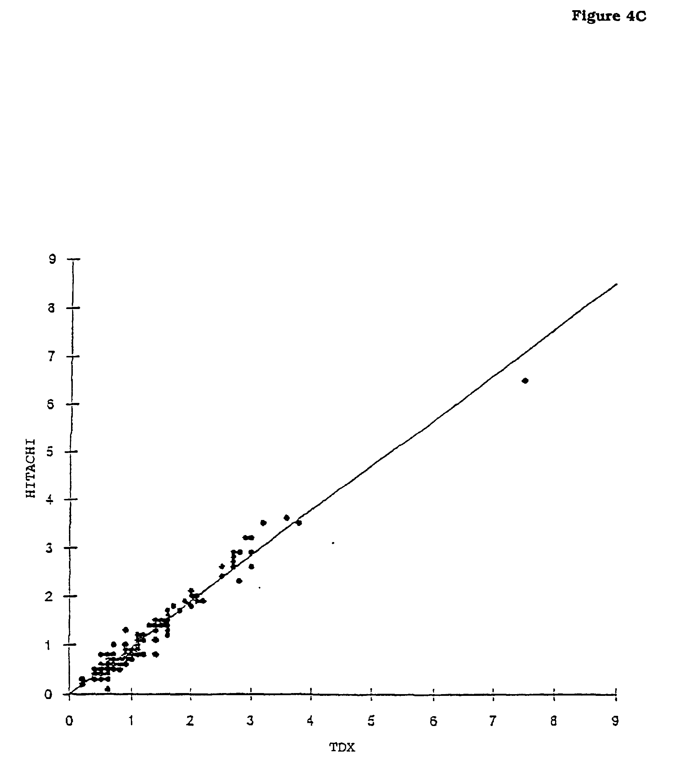

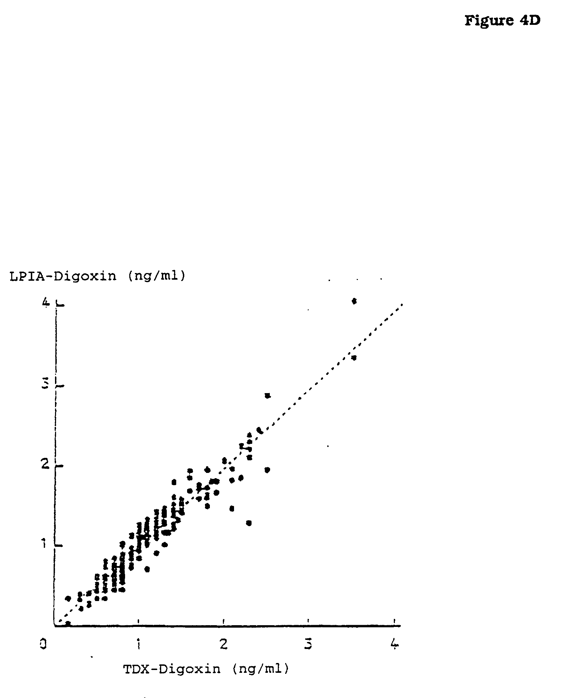

Patient serum specimens previously evaluated with TDₓ (Abbot) for digoxin concentration were tested using the above microparticle reagent and buffer reagent in a latex agglutination inhibition assay using the four instruments described above. The data obtained with the four above-described instruments was plotted against the results obtained by the TDₓ method and analyzed. Figure 4a shows the results obtained with COBAS FARA instrument. Figure 4b shows the results obtained with the COBAS MIRA instrument. Figure 4c shows the results obtained with the Hitachi 704 instrument. Figure 4d shows the results obtained with of LPIA-100 instrument.

As Figures 4a-d show, the latex agglutination inhibition assay conducted with reagents according to the present invention provide excellent results. The results obtained with the present invention reagents are clearly as good as, or better than, results obtained with the TDₓ system.

### EXAMPLE 6

With the same systems described above, a precision study was conducted using BioRad quality control serum or Roche calibrators. As shown by the data in Table 3, the present invention reagents provide far better precision than the currently accepted standard of 10% Coefficient of Variation (C.V.) at 1 ng/ml.

**TABLE 3**

| | | | | | | |
|---|---|---|---|---|---|---|
| 10 Day Study | | | | | | |
| 3 Conc Values | | | | | | |
| n=10 Assays/Value/Day | | | | | | |

| | 0.5 ng/ml | | 2.0 ng/ml | | 4.0 ng/ml | |
|---|---|---|---|---|---|---|
| Run-to-Run: | X | CV% | X | CV% | X | CV% |
| Day 1 | 0.546 | 7.38 | 1.968 | 4.14 | 3.985 | 2.16 |
| Day 2 | 0.542 | 5.97 | 1.939 | 2.95 | 3.973 | 1.54 |
| Day 3 | 0.515 | 4.54 | 1.868 | 2.68 | 3.973 | 1.76 |
| Day 4 | 0.493 | 6.01 | 1.869 | 3.25 | 4.038 | 2.53 |
| Day 5 | 0.491 | 8.16 | 1.934 | 3.61 | 4.103 | 1.14 |
| Day 6 | 0.559 | 5.78 | 2.017 | 3.48 | 4.228 | 2.08 |
| Day 7 | 0.581 | 5.18 | 1.957 | 3.89 | 4.034 | 1.19 |
| Day 8 | 0.635 | 6.94 | 2.012 | 1.83 | 4.152 | 3.08 |
| Day 9 | 0.634 | 6.71 | 1.987 | 2.89 | 4.092 | 1.04 |
| Day 10 | 0.633 | 6.88 | 1.992 | 3.84 | 4.216 | 1.98 |
| Day-to-Day (10 Days) | 0.563 | 9.5 | 1.963 | 3.37 | 4.079 | 2.22 |

### EXAMPLE 7

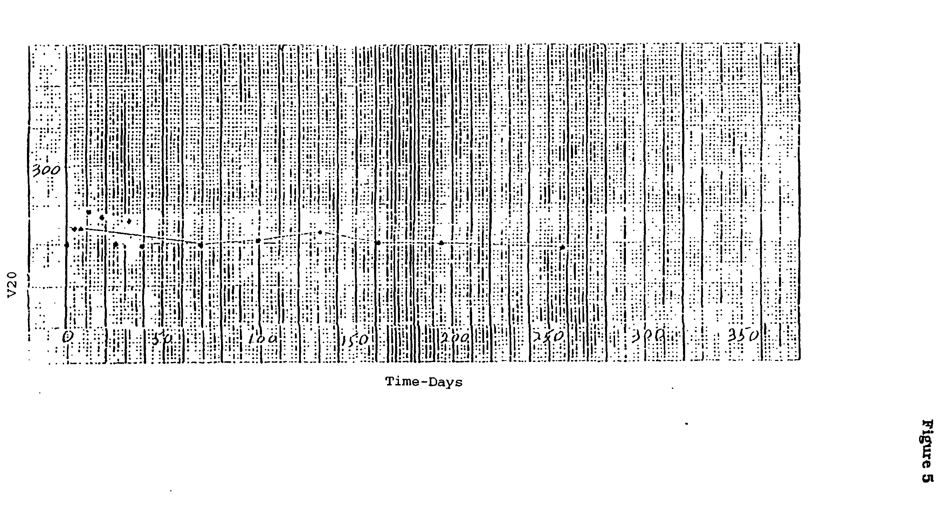

Using Seradyn's LPIA-100 system, the long term stability of the present invention reagents was evaluated. All reagents and calibrators were stored at 4°C. Measurements were conducted as follows: the serum sample and anti-digoxin antibody-containing buffer are incubated at 37° C for ten minutes. Upon completion of the incubation, a digoxin conjugated latex reagent is added to bind to unoccupied antibody binding sites. The rate of agglutination reaction is measured by the increase in absorbance at 950 nm and the reaction rate over a 5 minute absorbance measurement (V₂₀) was plotted versus time in days in Figure 5. As shown by the results in Figure 5, the reagents were stable for at least nine months.

### EXAMPLE 8

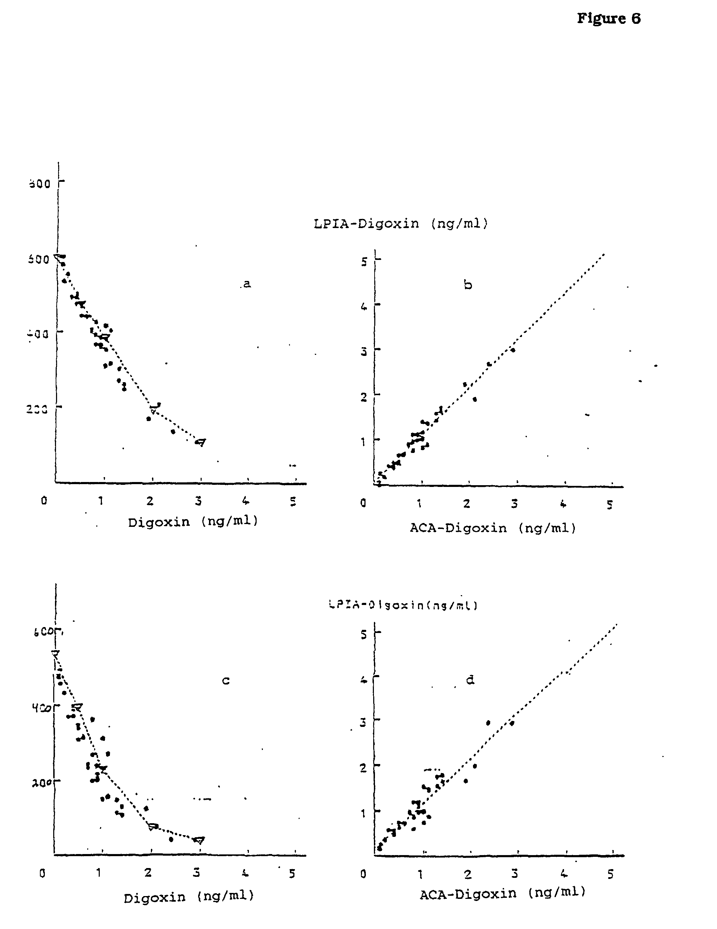

Digoxin latex-based microparticle reagents having diameters of 0.296 µm and 0.434 µm were prepared according to the above-described protocol. 35 patient samples previously evaluated for digoxin concentration by TDx were measured in a latex-agglutination inhibition assay using the LPIA-100 system. Figures 6a-6d, where Figures 6a and 6b refer to 0.296 µm particles and 6c and 6d refer to 0.434 µm particles, show the results.

Figures 6a and 6c are plots of LPIA-100 rate date versus digoxin concentration as determined by DuPont ACA®; Figures 6b and 6d are plots of sample digoxin concentration of the same samples as determined by the LPIA-100 system (y axis) versus DuPont ACA®.

### EXAMPLE 9

A digoxin microparticle reagent was prepared according to the protocol above but having a 0.184 µm diameter. Using the LPIA-100 instrument with a 20 µm sample volume and the following antibody buffer system:

| | |
|---|---|
| 0.9 or 3.5% | Sodium Chloride |
| 0 or 4% | Coline Chloride |
| 200 mM | Tris-(hydroxmethyl)amino methane, pH 7.5 |
| 1.2-1.3% | Sodium Dextransulfate |
| 1 mg/ml bovine | Serum albumin |
| 0.05% | Sodium azide |
| 1:40,000 | Diluted anti-digoxin-monoclonal antibody |

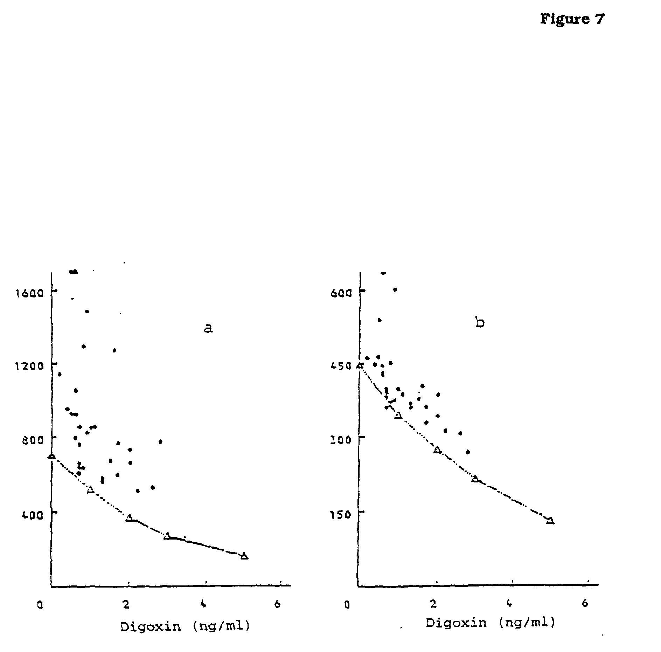

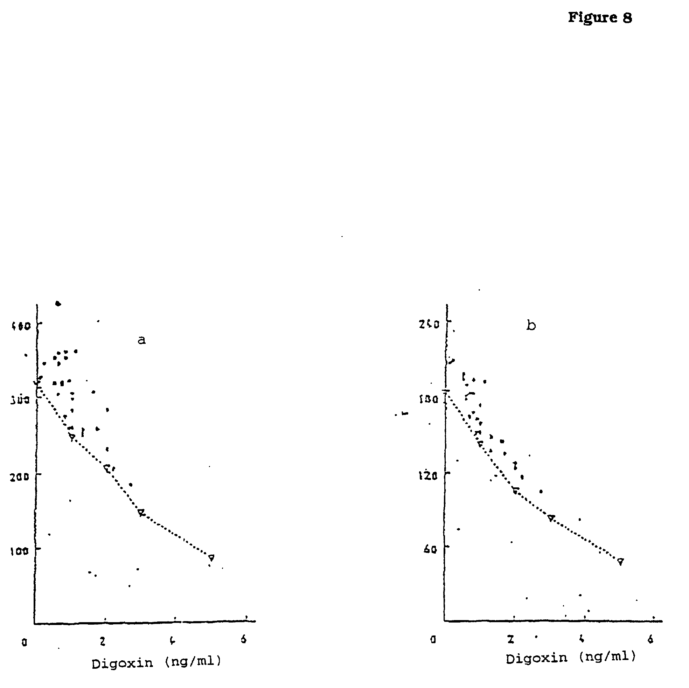

30 patient samples were evaluated for digoxin concentration. Figures 7a and 7b show the results obtained with 0.9 and 3.5% sodium chloride, respectively (using 0% choline chloride), and Figures 8a and 8b show the result obtained with 0 and 4% choline chloride, respectively, using 3.5% sodium chloride.

### EXAMPLE 10

A digoxin-sensitized microparticle reagent was prepared according to the above protocol but having a diameter of 0.292 µm and 35 patient samples were evaluated with the LPIA-100 system using 10 µl sample volumes. The antibody buffer composition used was follows:

| | |
|---|---|
| 3.5% | Sodium Chloride |
| 250 mM | Sodium acetate, pH 5.5-7.5 |
| 0.16% | Methyl cellulose |
| 0.1-4% | Fatty-acid-free-HSA |
| 250 mM | 1-ethyl-3-(3-dimethylaminopropyl-urea) |
| 0.05% | Sodium azide |
| 1:60,000 | Diluted anti-digoxin-monoclonal antibody |

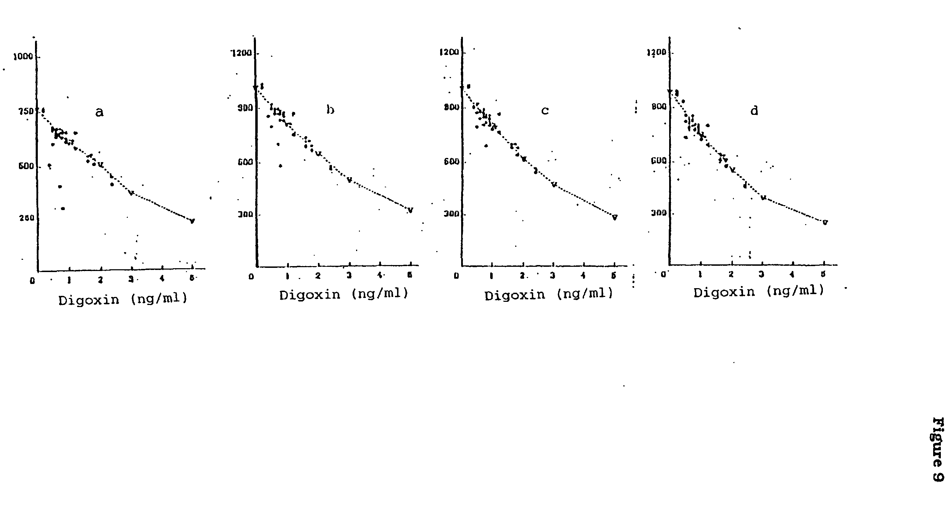

The results are presented in Figures 9 a-d where 9a is a control using 1.25 mg/ml FAF-HSA and with Figures 9b, 9c and 9d using 10, 20 and 40 mg/ml of FAF-HSA respectively.

## Claims

1. A sensitized microparticle comprising a carboxylate-modified latex particle and an immunoreactant covalently bound to said particle, wherein said particle has an overall diameter of from 0.18-0.6 µm and a surface carboxylate parking area of from 0.08-0.35 nm² (8-35 square angstroms). which is the surface area of said particle divided by the total number of carboxylic acid (-COON) and carboxylate (-COO⁻) functional groups on the surface thereof.

2. The microparticle of Claim 1, wherein said particle has a diameter of from 0.18-0.5 µm.

3. The microparticle of Claim 2, wherein said particle has a parking area of from 0.10-0.30 nm² (10-30 square angstroms).

4. The microparticle of Claim 1, wherein said immunoreactant is a hapten-protein conjugate.

5. The microparticle of Claim 4, wherein said immunoreactant is a digoxin-protein conjugate.

6. The microparticle of Claim 3, wherein said immunoreactant is a digoxin-human serum albumin conjugate.

7. The microparticle of Claim 1, wherein said immunoreactant is covalently bound to said carboxylate-modified latex particle in a condensation reaction in the presence of a carbodiimide.

8. The microparticle of Claim 7, wherein said carbodiimide is 1-ethyl-3-(3-dimethylaminopropyl)-carbodiimide hydrochloride.

9. The microparticle of Claim 7, wherein said condensation reaction is stopped with a compound selectded from the group consisting of ethanolamine, ethylenediamine, glucoseamine, glycine and lysine.

10. The microparticle of Claim 7 wherein said condensation reaction is stopped with glycine.

11. A particle-based immunoassay method comprising contacting the sensitized microparticle of Claim 1 with a complementary immunoreactant.

12. A kit comprising two components A and B, component A comprising the microparticle of Claim 1, component B comprising a buffer composition comprising a buffering agent, sodium chloride, choline chloride, a polysaccharide and fatty-acid-free serum albumin.

13. The kit of Claim 12, wherein said buffer composition further comprises a non-specific reaction suppressor of the formula: 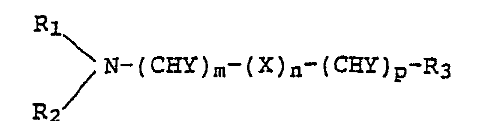 wherein
X is -NH-(CO)-NH-, -NH-(CS)-NH-, 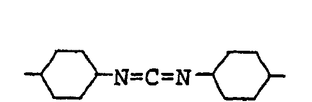 or -N=C=N-, R₁ and R₂, which may be the same or different, are C₁-C₅ linear or branched alkyl groups, or R₁ and R₂, together with nitrogen, is 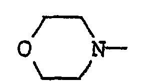 or the metho-p-toluenesulfonate salt thereof,
Y, which may be the same or different, is any of H, OH and halogen,
R₃ is -NR₁R₂, -NH₂, -CHY, cyclohexyl, or H,
m is an integer of from 0 to 5,
p is an integer of from 0 to 5, and
n is 0 or 1,
provided that at least one of m and p is at least 1 when n equals 1, and provided that when m=n=p=0, R₃ is H or -CH₂Y, and the acid addition salts thereof.

14. The kit of Claim 13, wherein said non-specific reaction suppressor is 1-ethyl-3-(3-dimethylaminopropyl)-urea.

15. The kit of Claim 12, wherein said buffer composition further comprises water, 1.0-5.0% by wt. sodium chloride, 1-15% by weight choline chloride, 0.2-0.3% by weight sodium dextransulfate or 0.05-1.0% by weight methyl cellulose, said percentages by wt. based on the total weight of the buffer, and 5-100 mg/ml of fatty-acid-free human serum albumin based on the total volume of the buffer.

16. The kit of Claim 15, wherein said buffer further comprises a complementary immunoreactant.

17. The kit according to Claim 16, wherein said complimentary immunoreactant is an anti-digoxin monoclonal antibody.

18. An immunoassay method comprising the steps of:
mixing a sample comprising an analyte to be detected with a buffer composition comprising water, a buffering agent, sodium chloride, choline chloride, a polysaccharide, fatty-acid-free serum albumin, and, optionally, a non-specific reaction suppressor of the formula: 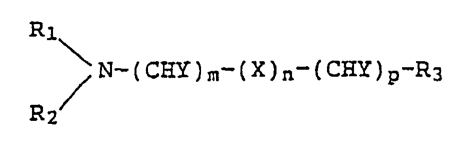 wherein
X is -NH-(CO)-NH-, -NH-(CS)-NH-, 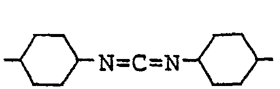 or -N=C=N-, R₁ and R₂, which may be the same or different, are C₁-C₅ linear or branched alkyl groups, or R₁ and R₂, together with nitrogen, is 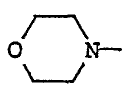 or the metho-p-toluenesulfonate salt thereof,
Y, which may be the same or different, is any of H, OH and halogen,
R₃ is -NR₁R₂, -NH₂, -CHY, cyclohexyl, or H,
m is an integer of from 0 to 5,
p is an integer of from 0 to 5, and
n is 0 or 1,
provided that at least one of m and p is at least 1 when n equals 1, and provided that when m=n=p=0, R₃ is H or -CH₂Y, and the acid addition salts thereof to produce a buffer-sample mixture, and
contacting said buffer-sample mixture with the microparticle of Claim 1.

19. The method of Claim 18, wherein said buffer further comprises a complimentary immunoreactant.

20. The method of Claim 18, wherein said buffer composition comprises 1.0-5.0% by wt. sodium chloride, 1-15% by weight choline chloride, 0.2-3.0% by weight sodium dextransulfate or 0.05-1.0% by weight methyl cellulose, said percentages by wt. based on the total weight of the buffer, and 5-100 mg/ml of fatty-acid-free human serum albumin based on the total volume of the buffer.

21. The method of Claim 19, wherein said buffer composition comprises 1.5-4.5% by wt. sodium chloride, 2-12% by weight choline chloride, 0.6-2.0% by weight sodium dextransulfate or 0.1-0.4% by weight methyl cellulose, said percentages by wt. based on the total weight of the buffer, and 60-75 mg/ml of fatty-acid-free human serum albumin.

## Patentansprüche

1. Sensibilisiertes Mikroteilchen, umfassend ein Carboxylat-modifiziertes Latexteilchen und einen kovalent an das Teilchen gebundenen Immunoreaktanten, wobei das Teilchen einen Gesamtdurchmesser von 0,18-0,6 µm und eine Oberflächen-Carboxylatparkfläche von 0,08-0,35 nm² (8-35 Square Angströms) aufweist. bei der es sich um die Oberfläche des Teilchens, dividiert durch die Gesamtanzahl an funktionellen Carbonsäure (-COOH) und Carboxylat (-COO⁻)-Gruppen auf dessen Oberfläche handelt.

2. Mikroteilchen nach Anspruch 1, wobei das Teilchen einen Durchmesser von 0,18-0,5 µm aufweist.

3. Mikroteilchen nach Anspruch 2, wobei das Teilchen eine Parkfläche von 0,10-0,30 nm² (10-30 Square Angströms) aufweist.

4. Mikroteilchen nach Anspruch 1, wobei der Immunoreaktant ein Hapten-Protein-Konjugat ist.

5. Mikroteilchen nach Anspruch 4, wobei der Immunoreaktant ein Digoxin-Protein-Konjugat ist.

6. Mikroteilchen nach Anspruch 3, wobei der Immunoreaktant ein Digoxin-Humanserumalbumin-Konjugat ist.

7. Mikroteilchen nach Anspruch 1, wobei der Immunoreaktant kovalent an das Carboxylat-modifizierte Latexteilchen in einer Kondensationsreaktion in Gegenwart eines Carbodiimids gebunden ist.

8. Mikroteilchen nach Anspruch 7, wobei das Carbodiimid 1-Ethyl-3-(3-dimethylaminopropyl)-carbodiimidhydrochlorid ist.

9. Mikroteilchen nach Anspruch 7, wobei die Kondensationsreaktion gestoppt wird mit einer Verbindung, gewählt aus der Ethanolamin. Ethylendiamin, Glucosamin. Glycin und Lysin umfassenden Gruppe.

10. Mikroteilchen nach Anspruch 7, wobei die Kondensationsreaktion mit Glycin gestoppt wird.

11. Auf einem Teilchen basierende Immunoassaymethode, umfassend das Kontaktieren des sensibilisierten Mikroteilchens nach Anspruch 1 mit einem komplementären Immunoreaktanten.

12. Kit, umfassend zwei Komponenten A und B, wobei die Komponente A das Mikroteilchen nach Anspruch 1 umfaßt, und die Komponente B eine Pufferzusammensetzung umfaßt, welche ein Puffermittel, Natriumchlorid, Cholinchlorid, ein Polysaccharid und ein fettsäurefreies Serumalbumin umfaßt.

13. Kit nach Anspruch 12, wobei die Pufferzusammensetzung weiterhin einen nichtspezifischen Reaktionssuppressor der Formel umfaßt: 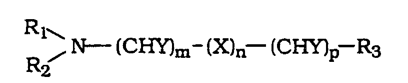 worin
X -NH-(CO)-NH-, -NH-(CS)-NH-, 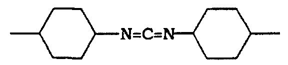 oder -N=C=N- ist. R₁ und R₂, welche gleich oder verschieden sein können, lineare oder verzweigte C₁-C₅-Alkylgruppen sind, oder R₁ und R₂ zusammen mit dem Stickstoff 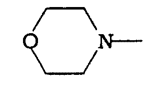 ist
oder das Metho-p-toluolsulfonatsalz hiervon,
Y, das gleich oder verschieden sein kann, irgendeines aus H, OH und Halogen ist,
R₃ -NR₁R₂, -NH₂, -CHY, Cyclohexyl oder H ist,
m eine ganze Zahl von 0 bis 5 ist,
p eine ganze Zahl von 0 bis 5 ist, und
n 0 oder 1 ist,
vorausgesetzt, dass mindestens eines von m und p mindestens 1 ist, wenn n gleich 1 ist, und vorausgesetzt, dass wenn m=n=p=0, R₃ H oder -CH₂Y ist, und die Säureadditionssalze hiervon.

14. Kit nach Anspruch 13, wobei der nichtspezifische Reaktionssuppressor 1-Ethyl-3-(3-dimethylaminopropyl)-harnstoff ist.

15. Kit nach Anspruch 12, wobei die Pufferzusammensetzung weiterhin Wasser, 1,0-5,0 Gew.-% Natriumchlorid, 1-15 Gew.-% Cholinchlorid, 0,2-0,3 Gew.-% Natriumdextransufat oder 0,05-1,0 Gew.-% Methylcellulose, wobei die Gewichtsprozentangaben auf das Gesamtgewicht des Puffers bezogen sind, und 5-100 mg/ml fettsäurefreies Humanserumalbumin umfaßt, bezogen auf das Gesamtvolumen des Puffers.

16. Kit nach Anspruch 15, wobei der Puffer weiterhin einen komplementären Immumoreaktanten umfaßt.

17. Kit nach Anspruch 16, wobei der komplementäre Immunoreaktant ein monoclonaler Antidigoxin-Antikörper ist.

18. Immunoassaymethode, umfassend die Schritte:
Mischen einer Probe, umfassend einen zu bestimmenden Analyten, mit einer Pufferzusammensetzung, umfassend Wasser, ein Puffermittel, Natriumchlorid, Cholinchlorid, ein Polysaccharid. fettsäurefreies Serumalbumin und wahlweise einen nichtspezifischen Reaktionssuppressor der Formel 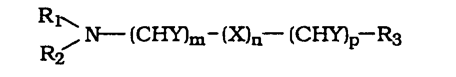 worin
X -NH-(CO)-NH-, -NH-(CS)-NH- 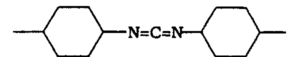 oder -N=C=N- ist, R₁ und R₂, welche gleich oder verschieden sein können, lineare oder verzweigte C₁-C₅-Alkylgruppen sind, oder R₁ und R₂ zusammen mit dem Stickstoff 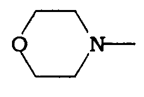 ist
oder das Metho-p-toluolsulfonatsalz hiervon,
Y, das gleich oder verschieden sein kann, irgendeines aus H, OH und Halogen Ist,
R₃ -NR₁R₂, -NH₂, -CHY, Cyclohexyl oder H ist,
m eine ganze Zahl von 0 bis 5 ist,
p eine ganze Zahl von 0 bis 5 ist, und
n 0 oder 1 ist,
vorausgesetzt, dass mindestens eines von m und p mindestens 1 ist, wenn n gleich 1 ist, und vorausgesetzt, dass wenn m=n=p=0, R₃ H oder -CH₂Y ist, und die Säureadditionssalze hiervon, um eine Puffer-Proben-Mischung zu erzeugen, und
Kontaktieren der Puffer-Proben-Mischung mit dem Mikroteilchen nach Anspruch 1.

19. Methode nach Anspruch 18, wobei der Puffer weiterhin einen komplementären Immunoreaktanten umfaßt.

20. Methode nach Anspruch 18, wobei die Pufferzusammensetzung 1,0-5,0 Gew.-% Natriumchlorid, 1-15 Gew.-% Cholinchlorid. 0,2-3,0 Gew.-% Natriumdextransulfat oder 0,05-1,0 Gew.-% Methylcellulose, wobei die Gewichtsprozentangaben auf das Gesamtgewicht des Puffers bezogen sind, und 5-100 mg/ml fettsäurefreies Humanserumalbumin umfaßt, bezogen auf das Gesamtvolumen des Puffers.

21. Methode nach Anspruch 19, wobei die Pufferzusammensetzung 1,5-4,5 Gew.-% Natriumchlorid, 2-12 Gew.-% Cholinchlorid, 0,6-2,0 Gew.-% Natriumdextransulfat oder 0,1-0,4 Gew.-% Methylcellulose, wobei die Gewichtsprozentangaben auf das Gesamtgewicht des Puffers bezogen sind, und 60-75 mg/ml fettsäurefreies Humanserumalbumin umfaßt.

## Revendications

1. Micro-particule sensibilisée comprenant une particule en latex modifiée avec des carboxylates et un réactif immunologique lié de manière covalente à ladite particule, **caractérisée en ce que** ladite particule à un diamètre total allant de 0,18 à 0,6 µm et une aire de couverture en surface carboxylate allant de 0,08 à 0,35 nm² (8 à 35 angströms au carré), correspondant à l'aire de surface de ladite particule divisée par le nombre total de groupements fonctionnels d'acide carboxylique (-COOH) et de carboxylates (-COO⁻) sur la surface de celle-ci.

2. Micro-particule selon la revendication 1, **caractérisée en ce que** ladite particule présente un diamètre allant de 0,18 à 0,5 µm.

3. Micro-particule selon la revendication 2, **caractérisée en ce que** ladite particule à une aire de couverture allant de 0,10 à 0,30 nm² (10 à 30 angströms au carré).

4. Micro-particule selon la revendication 1, **caractérisée en ce que** ledit réactif immunologique est un conjugué hapten-protéine.

5. Micro-particule selon la revendication 4, **caractérisée en ce que** ledit réactif immunologique est un conjugué digoxine-protéine.

6. Micro-particule selon la revendication 3, **caractérisée en ce que** ledit réactif immunologique est un conjugué digoxine-albumine de sérum humaine.

7. Micro-particule selon la revendication 1, **caractérisée en ce que** ledit réactif immunologique est lié de manière covalente à ladite particule en latex modifiée avec des carboxylates dans une réaction de condensation en présence de carbodiimide.

8. Micro-particule selon la revendication 7, **caractérisée en ce que** ledit carbodiimide est le chlorhydrate de 1-éthyl-3-(3-diméthylaminopropyl)-carbodiimide.

9. Micro-particule selon la revendication 7, **caractérisée en ce que** ladite réaction de condensation est arrêtée avec un composé choisi dans le groupe constitué d'éthanolamine, d'éthylènediamine, de glucoseamine, de glycine et de lysine.

10. Micro-particule selon la revendication 7, **caractérisé en ce que** la réaction de condensation est arrêtée avec de la glycine.

11. Procédé d'essais immunologiques à base de particule, **caractérisé par** la mise en contact de la micro-particule sensibilisée de la revendication 1 avec un réactif immunologique complémentaire.

12. Kit, **caractérisé en ce qu'**il comprend deux composants A et B, le composant A comprenant la micro-particule de la revendication 1, le composant B comprenant une composition tampon comprenant un agent tampon, du chlorure de sodium, du chlorure de choline, un polysaccharide et une albumine de sérum exempte d'acide gras.

13. Kit selon la revendication 12, **caractérisé en ce que** la composition tampon comprend en outre un agent de suppression de réaction non spécifique de formule : 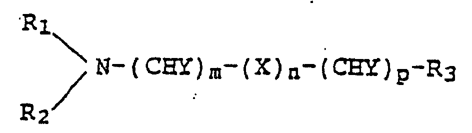 dans laquelle
X représente -NH-(CO)-NH-, -NH-(CS)-NH-, 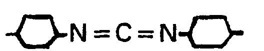 ou -N=C=N-, R₁ et R₂, qui peuvent être identiques ou différents, sont des groupements alkyles linéaires ou ramifiés en C₁-C₅, ou R₁ et R₂, avec de l'azote, représentent 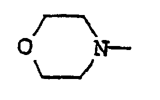 ou le sel de métho-p-toluènesulfonate de celui-ci,
Y, qui peut être identique ou différent, est l'un quelconque parmi H, OH et un halogène,
R₃ représente -NR₁R₂, -NH₂, -CHY, cyclohéxyle, ou H,
m est un nombre entier de 0 à 5,
p est un nombre entier de 0 à 5, et
n est 0 ou 1,
avec au moins l'un parmi m et p égal à au moins 1 lorsque n est égal à 1, et avec, lorsque m = n = p = 0, R₃ représentant H ou -CH₂Y, et les sels d'adition acide de ceux-ci.

14. Kit selon la revendication 13, **caractérisé en ce que** l'agent de suppression de réaction non spécifique est le 1-éthyl-3-(3-diméthylaminopropyle)-urée.

15. Kit selon la revendication 12, **caractérisé en ce que** ladite composition tampon comprend en outre de l'eau, 1,0-5,0% en poids de chlorure de sodium, 1-15% en poids de chlorure de choline, 0,2-0,3% en poids de dextransulfate de sodium ou 0,05-1,0% en poids de méthyl-cellulose, lesdits pourcentages en poids étant basés sur le poids total de tampon, et 5-100 mg/ml d'albumine de sérum humaine exempte d'acide gras basé sur le volume total de tampon.

16. Kit selon la revendication 15, **caractérisé en ce que** ledit tampon comprend en outre un réactif immunologique complémentaire.

17. Kit selon la revendication 16, **caractérisé en ce que** ledit réactif immunologique complémentaire est un anticorps monoclonal anti-digoxine.

18. Procédé d'essai immunologique comprenant les étapes consistant :
- à mélanger un échantillon comprenant un analyte à détecter avec une composition tampon comprenant de l'eau, un agent tampon, du chlorure de sodium, du chlorure de choline, un polysaccharide, une albumine de sérum exempte d'acide gras libre, et, optionnellement, un agent de suppression de réaction spécifique de formule : 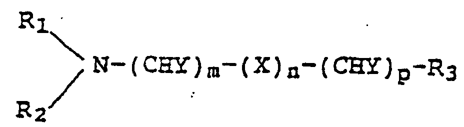 dans laquelle
X représente -NH-(CO)-NH-, -NH-(CS)-NH-, 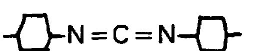 ou -N=C=N-, R₁ et R₂, qui peuvent être identiques ou différents, étant des groupements alkyles linéaires ou ramifiés en C₁-C₅, ou R₁ et R₂, avec de l'azote, représentent 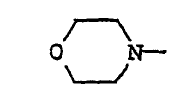 ou le sel de métho-p-toluènesulfonate de celui-ci,
Y, qui peut être identique ou différent, est l'un quelconque parmi H, OH et un halogène,
R₃ représente -NR₁R₂, -NH₂, -CHY, cyclohéxyle, ou H,
m est un nombre entier de 0 à 5,
p est un nombre entier de 0 à 5, et
n est 0 ou 1,
avec au moins l'un parmi m et p égal à au moins 1 lorsque n est égal à 1, et avec, lorsque m = n = p = 0, R₃ étant H ou -CH₂Y, et les sels d'adition acide de ceux-ci pour fabriquer un mélange tampon-échantillon, et
à mettre en contact ledit mélange tampon-échantillon avec la microparticule selon la revendication 1.

19. Procédé selon la revendication 18, **caractérisé en ce que** ledit tampon comprend en outre un réactif immunologique complémentaire.

20. Procédé selon la revendication 18, **caractérisé en ce que** ladite composition tampon comprend 1,0-5,0% en poids de chlorure de sodium, 1-15% en poids de chlorure de choline, 0,2-3,0% en poids de dextransulfate de sodium ou 0,05-1,0% en poids de cellulose méthylée, lesdits pourcentages en poids étant basés sur le poids total de tampon, et 5-100 mg/ml d'albumine de sérum humaine exempte d'acide gras basé sur le volume total de tampon.

21. Procédé selon la revendication 19, **caractérisé en ce que** ladite composition tampon comprend 1,5-4,5% en poids de chlorure de sodium, 2-12% en poids de chlorure de choline, 0,6-2,0% en poids de dextransulfate de sodium ou 0,1-0,4% en poids de cellulose méthylée, lesdits pourcentages en poids étant basés sur le poids total de tampon, et 60-75 mg/ml d'albumine de sérum humaine exempte d'acide gras.
